# EUROPEAN PATENT SPECIFICATION

(11) **EP 0 664 133 B1**
(45) Date of publication and mention of the grant of the patent: **23.09.1998**
(21) Application number: 94923110.4
(22) Date of filing: 14.07.1994
(51) Int. Cl.: A61L 27/00, A61K 6/033

(54) **PREPARATION "OSTIM APATITE" FOR STIMULATING GROWTH IN BONE TISSUE**
OSTIM APATIT PRÄPARAT ZUR WACHSTUMSFÖRDERUNG IM KNOCHENGEWEBE
PREPARATION D'"OSTIM APATITE" STIMULANT LA CROISSANCE DES TISSUS OSSEUX

(30) Priority: 21.07.1993 RU 93037113
(43) Date of publication of application: 26.07.1995
(73) Proprietor: AKTSIONERNOE OBSCHESTVO ZAKRYTOGO TIPA "OSTIM", Moscow, 119034 (RU)
(72) Inventor: RUDIN, Vsevolod Nikolaevich, Moscow, 125502 (RU); BOZHEVOLNOV, Viktor Evgenievich, Moscow, 109004 (RU); ZUEV, Vladislav Petrovich, Moscow, 123425 (RU); KOMAROV, Vladimir Fedorovich, Moscow, 115582 (RU); MELIKHOV, Igor Vitalievich, Moscow, 117331 (RU); MINAEV, Vladimir Vasilievich, Moscow, 121248 (RU); ORLOV, Andrei Yurievich, Moscow, 121357 (RU); PANKRATOV, Aleksandr Sergeevich, Moscow, 113941 (RU); DREVAL, Anatoly Alexandrovich, Moscow, 114020 (RU)
(74) Representative: Blumbach, Kramer & Partner GbR
(86) International application number: RU9400151
(87) International publication number: WO9503074

(56) References cited:
- EP-A- 0 278 583
- EP-A- 0 486 813
- WO-A-89/10896
- US-A- 4 548 959
- US-A- 5 009 898

## Description

### Field of the Invention

The invention relates to medicine and, more particularly, the invention relates to reconstructive bone surgery, surgical stomatology, traumatology and orthopaedics.

### Prior Art

One of the most important task, when producing a preparation to used in reconstructive surgery, is to produce a synthetic substance whose chemical composition and crystal parameters are identical to the basic substance - calcium orthophosphate being a basic inorganic component of the tissue of living organisms. For this purpose, synthetic hydroxy apatite with a chemical formula Ca₅(PO₄)₃OH or Ca₁₀(PO₄)₆(OH)₂ has found world-wide practical application.

However, various preparations of hydroxy apatite have a different degree of osteoinductive potential due to a different rate of dissolution and bioresorption determined by the hydroxy apatite grain size, the specific surface, density and microporosity.

The known and presently used preparations feature rather a high percentage of after-operation complications in the form of muscosa necrosis and delayed healing, and this resulted in development of other multicomponent compounds based on hydroxy apatite.

Known in the art is a composition based on hydroxy apatite for creating an artificial bone tissue (USSR Inventor's Certificate No. 1,005,344, A16 K 33/06, 1986). The composition includes hydroxy apatite or beta-tricalcium phosphate, gelatin, catgut and water. To prepare this composition, natural mineral salts are used. The required porosity is provided using a finely ground dry catgut. After special treatment an artificial tissue is obtained having a preset size and shape, which in a living organism is gradually replaced by a newly formed bone tissue.

However, the use of this preparation leads to a high percentage of postoperative complications due to a different rate of dissolving and bioresorption.

The closest to the proposed preparation in technical essence and the result obtained is a means for stimulating growth of bone tissue based on hydroxy apatite with a grain size of 10-1.0 µm (B. Alliot-Light et al-Cellular activity of osteoblasts in the presence of hydroxy apatite. Biomate-rials, 1991, vol.12. pp. 752-756)

This preparation is disadvantageous in weak biochemical activity due to the fact that the hydroxy apatite grains are rather big. It has been proved experimentally that the preparation stimulates the growth of fibroblasts but do not stimulate the growth of bone tissue cells (osteoblasts).

### Disclosure of the invention

The basic object of the invention is to provide a preparation stimulating the process of reparative osteogenesis having a high biological and specific pharmacological activity.

This object is attained by using the claimed preparation "OSTIM APATITE" for stimulating growth in bone tissue consisting of an aqueous pacts of hydroxy apatite with a concentration of 18-36% and with a particle size of 0.015-0.06 µm.

The preparation reacts to variations of the ambient biochemical medium; for example, a decrease of pH results in dissolving providing active utilization of ions of Ca and PO₄ in the process of osteogenesis: the side and shape of the crystals are maximally adapted to the bone tissue structure so that they can be used in the osteoreparative process as a building material without additional dissolving in the organism. The preparation has high sorption activity with respect to protein and amino acids.

The preparation authenticity is determined by the qualitative and quantitative analysis for water, ions of Ca and PO₄.

The conducted toxicological study has shown that the tested modification of the preparation is practically nontoxic compound, and this is demonstrated by hypodermic, intramuscular and parenteral injection of the preparation in the organism of experimental animals. The preparation has no irritating action on the skin and mucosas, does not penetrate through the skin covers, has no sensibilization properties. The introduction of the preparation in maximum doses (intramuscular - 3.14; hypodermic - 5.0; parenteral - 5.54 g/kg of the animal weight) did not cause its death during 2 weeks of observation. The general condition and behavior of the experimental animals subjected to the hypodermic and intramuscular injection into their organism were the same as the behavior of the control animals. After the parenteral injection of the preparation into the abdominal cavity, during the first week of observation the animals were stiff and had a rumpled hair cover. The recording of the body weight of the experimental animals revealed no significant changes. The activity of the liver ferments in both comparative groups was essentially the same. The pathological anatomic dissection of the experimental animals has found no any disturbances in the mactrostructure of the internal organs. In the experiments to study acute toxicity on white mice, rats, guinea-pigs and rabbits it was found that the preparation has no gender or sexual sensitivity.

In the study of chronic toxicity of the preparation it was found that its injection in the maximum possible doses (intramuscular - 2.8; hypodermic - 2.2; parenteral - 4 g/kg of the animal weight) has no effect on the functions of the main systems of the organism. The studied characteristics of the nervous system: summary-threshold index, latent period and number of peeps out from a dark chambers with holes, vertical and horizontal activity and peeps out in the "open area" did not change compared to the control values. The liver activity, daily diuresis, content of protein and chlorides in the urea of the experimental animals did not change compared to the control animals, and this indicates to the absence of harmful effect of the preparation on the liver and kidneys. The amount of leukocytes, erythrocytes and hemoglobin in the peripheral blood of the experimental animals remained at the control value level. The pathological anatomic dissection of the experimental animals has found no any disturbances in the mactrostructure of the internal organs. Their mass factors were the same as those of the control animals.

All these characteristics were the same during 6 months of the experimental injections.

The study of specific types of toxicity of the preparation has shown the absence of embriotoxic activity and effect on the postanal development of the offspring, when the preparation was injected hypodermically to pregnant rats in an amount of 1000 mg/kg of weight; the study of the effect of the preparation on the male reproductive function proved the absence of any negative effect. The performed studies have shown than the preparation has no effect on the reactions of the cellular and hummoral immunity and also has no allergic activity. The investigation of the mutagenic activity of the preparation included study of chromosomal aberrations in the osseous cerebrum cells, dominant lethal mutations in the embryonic cells of mice, ability to induce genic mutations on the indicator bacteria in the Eims tests. In the course of operation no mutagenic activity of the preparation was found.

Therefore, the experimental study has demonstrated that this preparation is practically non-toxic with different ways of entering the organism and no side effects have been found. The injection of high doses (1-6 g/kg) also had no negative effects so that the preparation has no contraindications to its use as a medicine.

The study of the specific pharmacological activity of the preparation consisting in stimulating the osteoreparation processes was carried out by comparing the proliferative activity of the osteoblasts in the cellular culture in the presence of the preparation and in the control.

To obtain the primary culture of osteoblasts, use was made of newborn daily baby rats which were killed by dislocating the cervical vertebras. The cellular culture was obtained using classical methods. 7 days after the cultiva-tion, a bunch of appendage cells came onto the surface and form a monolayer around the tissue fragments. The shape of the cells corresponded to the picture of osteoblasts shown in the medical literature. Before the experiments the viability of the osteoblasts was verified by means of a trypan blue vital dye. The microscopic analysis has shown that the cell viability is 85-90%, i.e. corresponding to the permissible limits.

The cell suspension in a concentration of 10⁵ cells/ml was put in doses of 1.5 ml into glass flasks for cultivation. The preparation was added to the test samples to get a final concentration of 0.1 or 1.0 mg/ml. The proliferative activity of the osteoblasts was estimated using the radio indicator method by introducing ³H-thymidine into the DNA of the cells (β-radiometer "Beta-2").

The study has shown that the preparation stimulates the proliferation of the osteoblasts in the cell culture increasing the number of cells and intensity of the marked predecessor of the DNA- ³H-thymidine compared to the control by 40-100%.

In addition, we studied the effect of the preparation on the activity of the marker ferment of the osteoblasts (alkali phosphatase), the level of the calcium ions and intensity of peroxide oxidation of the lipides in the rat blood serum.

The preparation increases the alkali phosphatase activity only when it is injected into the abdominal cavity in a dose of 5.4 g/kg (which is not supposed to be done under clinical conditions) and does not change this activity at hypodermic and intramuscular injection. This confirms the absence of a system inflammatory reaction of the organism to the preparation and manifests its low toxicity. When treating a mandible, the activity of this serum increases. The preparation has no effect on the peroxide oxidation of lipides in the blood serum of the intact rats and reduced to a normal value the increased level of the peroxide oxidation in the animals with an injured mandible at the fourth day after the injury. Regardless of the methods of injection, the preparation has no effect on the concentration of the calcium ions in the blood of the healthy rats and those with experimentally broken mandible.

Therefore the data obtained enable us to consider this preparation as a stimulator of the profilerative and biosynthetic activity of the osteoblasts.

The morphological studies were effected on the tissue reaction to the implantation of hydroxy apatite in the experiments with rats having a damaged mandible. To this end, a group of ten experimental animals and ten control animals were treated under Nembutal narcosis: using a dental drill, a channel 1.0 mm in diameter and 2 mm long was made in the region of the mandible alveolar appendage. The channel was oriented along the lower incisor to damage the structures of periosteum, bone, periodontium. The traumas of the control group animals were covered with a small amount of adhesive, the experimental group rats had their channel filled with 20% paste of hydroxy apatite.

The experiment were over after 4 days, 2.6 weeks and 6 months.

The macroscopic study of the acted-on area in both the control and experimental groups of animals demonstrated the absence of pronounced side effects even just after the operation. The intervention did not impede the reception of food by the animals, the epithalization of the damaged gingival zone developed without complications. Therefore, the range of the analyzed objects in the study was restricted by the connective structures of the regenerate area.

The processing of the obtained histological samples and making the preparations for the study through the optical and electronic scanning microscopy were effected using ordinary technique.

The results of this study demonstrated at the earlier date of observation in the experimental group a reduced inflammatory reaction compared to that of the control animals. In contrast to the control, in the experimental group of animals there were noted a much larger amount of fibrill producing cells which often form a cytoplasm network, the largest cisterns being located at the peripheral parts of the cytoplasm tightly filled with fine fibrous matter; the hyaloplasm appears in the form of osmiofilling layers. A combination of intensive fibrill production with processes of active reconstruction of the newly formed fibrous elements by means of collagenoclasts was also typical.

2 weeks after the beginning of the experiment, synthetic activity of the osteoblastic structures continued in the experimental group of animals. The second-type osteoblasts are registered as a basic cellular element, i.e. the mature, functionally active cells. The implant intergrows with connective tissue containing a vascular component, a vascular reaction is observed in the tissue adjoining the experimental action area.

The term of 1.5 months allowed us to discover in the basic group of experimental animals a next stage of reparative osteogenesis including ossifications processes as a main component. The events described for the earlier stages are completed. The superposition of these processes makes the osteoreparation pattern irregular, mosaic, because the zones of bone mature are close to the reaction of the fibrovascular tissue on the implant material. The structure of the newly formed bone becomes coarse. The areas of bone defect directly adjoining the implant regenerate much faster.

After 15 months the difference in the course of stimulated and non-stimulated osteogenesis in pure models are no longer observed.

Thus, from the analysis of the obtained data it is clear that implantation of hydroxy apatite manifests a pronounced stimulating action on the reparative osteogenesis process at its earlier stage.

Taking into account the positive characteristics of the preparation obtained in the course of preclinical study, the clinical tests were conducted on volunteers treated under stationary and dispensary conditions because of mandible fractures (36 persons), abscesses of jaw bones (7 persons), parodontium (12 persons), anastomosis of the oral cavity with the upper jaw antrum (2 persons), granulating and granulematose forms of periodontitis (9 persons), traumatic osteomyelitis of the low jaw (8 persons), fracture of long bones of limbs (5 persons), limb bone fracture (24 persons). The preparation was implanted after the patients had agreed to take part in the clinical study.

In the process of tests it was found that the preparation has no effect on the liver function, mineral exchange in the organism, does not change the general clinical laboratory characteristics compared to the control patients. During the injection the preparation causes no sickly feeling in the patients, causes no inflammatory reaction at the place of implantation that does not significantly prolong the time of the operation or complication of its technique. No allergic reactions to the preparation were noted. The nearest post-operation period was smooth for the majority of the patients in spite of the fact that in some cases the preparation was implanted after active inflammatory processes in the bone. Only in one case we observed a progress in the inflammatory process associated with the pathology character (bone tissue (actinomycosis).

To study the osteoreparative action of the preparation, immediately after its injection the intensity of mineralization of the bone in the damage zone was studied introducing radiopharmpreparations. The work was done observing the "Norms of Radiation Safety for Patients"; the study was conducted at the patients with broken low jaws in the control and basic groups. This study has shown that the implantation of the preparation into the fracture and pits of the removed teeth provides favorable conditions for the process of reparation of the bone tissue during the whole period of immobilization of the broken pieces.

Given below are example of clinical observations.

### Example 1.

Patient B. appealed to the clinic with complains for sickly swelling of the soft tissue in the mandible zone to the right, at level 76, pains in these teeth when taking a bit of food, rising a body temperature to 37.8°C. A diagnosis was as follows: radicular cyst in a suppuration phase. The osteoscintogramm obtained by intra-venous injection of radiopharmpreparations revealed an origin of increased accumulation of 127% relative to intact parts of the bone tissue indicating to an active inflammatory process in the bone. After preliminary course of antibacterial therapy, a cystectonomy operation was made with removal of the abscessed tooth. The formed bone cavity was filled with 33% paste of hydroxy apatite. During the nearest postoperational period no inflammatory phenomena were observed, the seams disappeared after 5-6th day, the operative wound mucosa acquired normal color.

### Example 2.

Patient D. A closed hip fracture; the femur was fixed with a pin. 5 days later the patient temperature risen to subfebrile level, inflammatory phenomena in the hip fracture zone followed by fistula with pussy discharge. The control X-ray photograph showed a picture of postoperational femur ostemyelitis without any sign of consolidation. A secondary operation was done for the patient with curet-tement and ultrasonic cavitation of the pussy cavity with antiseptic (0.02% solution of chlorohexydine) and antibiotic (Claforan). The pin was removed and the cavity at the fracture was filled with 18% paste of hydroxy apatite in an amount of 10 grams with addition of 1 gram of Claforan. The wound was stitched completely and drains were left in the cavity for irrigation with antibiotics and active lavage. Two weeks later the control X-ray picture showed formation of a primary periosteal callosity.

### Example 3.

Patient B was put into hospital with a fractured mandible in the corner area 6 hours after he had got this trauma. The broken pieces were immobilized using a jaw split and a tooth was removed from the fracture line. 35% paste of hydroxy apatite was injected through the pit of the removed tooth and the pit was closed with catgut. During the whole immobilizing period no inflammatory-exudate phenomena were noted. Allergic reactions were also not observed. The osteoscintigrams made on the 7th. 14th and 21st days after the trauma showed the following accumulation of radiopharmpreparation in the injury zone: 177, 155, 147% respectively (the calculation was made with respect to the symmetric part on the intact side).

### Example 4.

Patient K.- a mandible fracture in the corner area. The patient was taken to hospital within 1 day after the injury. No hydroxy apatite was implanted. The osteoscintigrams made on the 7th, 14 and 21st days in the injury zone showed the following accumulation of the radiopharmpreparation: 147, 216, 195% respectively.

From Example 3 and 4 it is evident that the degree of stress of the compensatory mechanisms of the mineral exchange in the patient, to whom the preparation was implanted, is lower. This confirms the fact that the preparation contributes to better physiological conditions for the bone tissue regeneration during the nearest postoperational period.

The preparation is obtained as follows: orthophosforic acid is added to an aqueous suspension of the calcium oxide with continuous stirring. With strict observance of the technology, 100% yield of crystalline hydroxy apatite is obtained as a final product.

In order to obtain a concentrated paste, the hydroxy apatite suspension is compacted in a centrifuge during 20-30 minutes. To obtain a low-concentration paste, the suspension is heated in a water bath during 3 hours and left for 10-12 hours for cooling and sedimentation of the hydroxy apatite. The mother solution is removed and the sediment is filtered.

The paste is packed into sealed flasks or plastic tubes and then is sterilized. The paste preserves the initial homogeneity, plasticity and dispersity during at least 2 years.

### Industrial applicability

The claimed preparation features high chemical purity corresponding to international standard A TM:F 1185-88 and has no other calcium phosphate compounds. The preparation is characterized by a high degree of dispersion and specific free surface:approximately 100 m²/g. In contrast to all such compounds known in the art,the preparation is not an osteoconnector, has pharmacological activity due to direct stimulation of the growth of bone tissue cells and features no side effects.The preparation is produced in a form convenient for clinical application.

## Claims

1. A preparation for stimulating growth in bone tissue based on hydroxy apatite, characterized in that the hydroxy apatite is taken in the form of an aqueous pasta with a concentration of 18-36% by weight with a particle size of 0.015 to 0.06 µm.

## Patentansprüche

1. Zubereitung zur Stimulierung des Wachstums im Knochengewebe basierend auf Hydroxylapatit dadurch gekennzeichnet, daß das Hydroxylapatit in der Form einer wässrigen Lösung mit einer Konzentration von 18 - 36 Gewichtsprozent mit einer Partikelgröße von 0,015 bis 0,06 µm verwendet ist.

## Revendications

1. Préparation pour stimuler la croissance des tissus osseux basée sur l'hydroxy-apatite, caractérisée par le fait que l'hydroxy-apatite est prise sous forme d'une pâte aqueuse avec une concentration de 18 à 36 % en masse avec une taille de particule de 0,015 à 0,06 µm.
